# EUROPEAN PATENT SPECIFICATION

(11) **EP 1 750 675 B1**
(45) Date of publication and mention of the grant of the patent: **29.10.2008**
(21) Application number: 04735713.2
(22) Date of filing: 02.06.2004
(51) Int. Cl.: A61K 9/20, A61K 9/16, A61K 31/485

(54) **STABLE ORAL PHARMACEUTICAL COMPOSITIONS OF BUPRENORPHINE AND SALT THEREOF**
STABILE ORALE PHARMAZEUTISCHE ZUSAMMENSETZUNGEN VON BUPRENORPHIN UND SALZ DAVON
COMPOSITIONS PHARMACEUTIQUES ORALES STABLES DE BUPRENORPHINE ET SEL DE CELUI-CI

(43) Date of publication of application: 14.02.2007
(73) Proprietor: Galephar M/F, 6900 Marche-en-Famenne (BE)
(72) Inventor: VANDERBIST, Francis, B-1650 Beersel (BE); SERENO, Antonio, B-1820 Melsbroek (BE); BAUDIER, Philippe, B-1180 Uccle (BE)
(74) Representative: Vandeberg, Marie-Paule L.G.
(86) International application number: PCT/BE2004/000081
(87) International publication number: WO 2005/117838

(56) References cited:
- WO-A-20/04000296
- US-B1- 6 365 596

## Description

The present invention describes stable oral compositions of buprenorphine or salt thereof. The stable compositions of buprenorphine are obtained by mean of addition of low concentration of an antioxidant and a chelating agent in the formulation. Particularly the molar ratio between the antioxidant and buprenorphine is strictly below 1:1. The invention further describes manufacturing processes to obtain said compositions.

### Background of the invention

Buprenorphine and buprenorphine hydrochloride are drugs which may be prepared from the alkaloid thebaïne. It is an opioid agonist/antagonist about forty times as potent as morphine. It is recognized as having lower dependence liability than other strong opioid analgesics and is indicated for the treatment of moderate to severe pain. It is also used in opioid addiction treatment.

Buprenorphine hydrochloride (CAS number 53152-21-9) is marketed in several countries and under various pharmaceutical forms, i.e. intramuscular and intravenous injection or sublingual tablets for treatment of pain at doses of 0.2 and 0.4 mg and at 2 and 8 mg for treatment of opioid dependence. Buprenorphine hydrochloride is an odourless white or off-white crystalline solid. The solubility of buprenorphine hydrochloride (20°C) is of about 20 mg/ml in water, 42 mg/ml in ethanol, 0.02 mg/ml in cyclohexane and of more than 500 mg/ml in glacial acetic acid.

Buprenorphine hydrochloride does not exhibit polymorphism. In terms of stability, buprenorphine hydrochloride is stable at high temperatures (up to 120°C) and in neutral solutions (pH 5-7). On the contrary, the molecule shows significant degradation in acid and alkaline conditions. But the parameter which most significantly affects buprenorphine's stability is oxidation, with the possibility of forming as many as 14 impurities, the main one being the 10-keto-buprenorphine

US patent 6,365,596 provides a first solution to stabilize oral pharmaceutical compositions of buprenorphine.

The solution proposed by US 6,365,596 is a pharmaceutical composition containing buprenorphine and at least one antioxidant in a molar ratio between said antioxidant and buprenorphine ranging from 1:1 to 3:1. The inventors also discovered that the stability of buprenorphine hydrochloride was improved when magnesium ion and polyvinylpyrrolidone were absent from the composition.

### Description of the invention

The present invention describes stable oral compositions of buprenorphine or salt thereof. Particularly, the composition of the present invention shows a particularly advantageous stability profile as well in terms of impurity profile as in terms of aspect (coloration of the tablets).

Oral compositions of buprenorphine and its salt are known to be particularly unstable. The main parameter provoking degradation of buprenorphine is oxidation, with the formation as principal impurity, of 10-keto-buprenorphine.

Buprenorphine sublingual tablets are currently marketed in different countries of the European Union, under different trademarks (Temgesic^{®}, Subutex^{®}, ...) and at different dosage strengths (0.2, 0.4, 2 and 8 mg).

The first attempts to manufacture stable oral compositions of buprenorphine HCl were made with the following formulation and result in a very unstable product (see table 1). As also shown in table 1, and surprisingly enough, the marketed reference treatment (Temgesic, Schering-Plough) also shows a significant degradation after respectively 19 and 17 months, i.e. before the expiration date which is of at least 24 months for this product. The batches of Temgesic^{®} 0.4 mg analyzed were purchased from a pharmacy and are therefore considered to have been stored at ambient temperatures. The qualitative compositions of Temgesic^{®} 0.4 mg and of in house batches of buprenorphine 0.4 mg were the same i.e. buprenorphine hydrochloride, lactose monohydrate, mannitol, corn starch, citric acid, sodium citrate, povidone and magnesium stearate. It should be noted that in the present invention, citric acid is used in the compositions as a pH modifier agent and not as an antioxidant (while it is sometimes described as such in galenic publications). The reason for this is that, as described by Valenti in US patent 6,365,596 and in the table hereinbelow, the weak antioxidant properties of citric acid are far from being powerful enough to stabilize buprenorphine as its salt in the oral composition.

**Table 1 : stability data of different oral compositions of buprenorphine hydrochloride stored at 25°C / 60 % relative humidity in aluminium/PVC blisters for in-house batches and in Aluminium / PVC /PVDC for Temgesic batches**

| | | | Buprenorphine | Total |
|---|---|---|---|---|
| product | Batch | Time after | | |
| | | | hydrochloride | impurities |
| (manufacturer) | Number | manufacturing | | |
| | | | content (%) | (%) |
| Temgesic 0.4 mg | 9009 | 19 months | 89.6 | 7.2 |
| (Schering-Plough) | 9010 | 17 months | 89.7 | 9.8 |
| | R102 | 12 months | 93.3 | 11.9 |
| in-house batches | | | | |
| | R105 | 12 months | 92.8 | 12.3 |
| (Galephar MF S.A.) | | | | |
| | R106 | 12 months | 90.0 | 12.6 |

Valenti and al had already discovered a way to stabilize oral compositions of buprenorphine or of its salts by adding an antioxidant in the composition in a molar ratio between said antioxidant and buprenorphine from 1:1 to 3:1. Following US patent 6,365,596, the preferred antioxidant is ascorbic acid. In the present invention, it has been surprisingly discovered that both the impurity profile of buprenorphine in oral forms, and especially in sublingual tablets, and the aspect of the tablets may be significantly improved by adding in the oral composition an antioxidant agent at low doses and a chelating agent, i.e. an agent able of sequestring traces of free cations like Mg⁺⁺, Fe⁺⁺, Ca⁺⁺, ...

The present invention also allows to obtain an optimal stabilization of buprenorphine or its salt in the oral composition using a lower concentration than the one described by Valenti in 6,365,596. The diminution of the amount of antioxidant in the oral composition containing buprenorphine allows to avoid the apparition of colored spots on the tablet's surface due to the degradation of the antioxidant. This phenomenon is particularly marked when the antioxidant agent used is ascorbic acid.

Also surprisingly, the combination of low amounts of antioxidants and the chelating agents in the buprenorphine composition allows to obtain a very stable composition even if the compositions further contain Magnesium cation and povidone. This is again in opposition to the content of US patent 6,365,596.
Consequently, the present invention relates to a stable composition of buprenorphine or salt thereof containing low concentrations of one antioxidant agents and a chelating agent. The antioxidant may be chosen, but is not limited to, ascorbic acid or salt thereof, derivatives of vitamin E, butylhydroxyanisole (BHA), butylhydroxytoluene (BHT), propylgallate, sodium bisulphite,...
The chelating agent may be chosen, but is not limited to edetic acid, malic acid,..
Advantageously, the molar ratio chelating agent(s)/antioxidant(s) is comprised between 0.05 and 20, preferably between 0.1 and 15, most preferably between 0.1 and 5.

The concentration of the chelating agent with respect with the total composition is advantageously between 0.01 % and 5 % (weight by weight), preferably between 0.05% and 3%.

The molar ratio between the antioxidant and buprenorphine is advantageously comprised between 1:1000 and 1:10, preferably between 1:100 and 1:5, most preferably between 1:100 and 1:1.5,
A particular aspect the present inventions is to allow to obtain stable composition of buprenorphine when magnesium stearate and povidone are used respectively as lubricant and binder. This is in contradiction to the discovery of US 6,365,596. The present invention also concerns compositions containing, in addition to buprenorphine, a second active ingredient which may be either an other analgesic drug either an opioid antagonist like naloxone.

The further examples and stability data will give a more comprehensive view of the invention.

### Example 1

Different formulations of buprenorphine HCl sublingual tablets were manufactured with different amounts of antioxidant agent (ascorbic acid and the presence or not of a chelating agent (EDTA).
The different formulations are given in table 2.

**TABLE 2 : quantitative compositions of buprenorphine HCl tablets**

| ***Ingredients*** | mg / tablet | | | | | |
|---|---|---|---|---|---|---|
| | | | | | | |
| | 20K | 21K | 22K | 25K | 26K | 27K |
| | 2002/2 | 2002/2 | 2002/2 | 2002/2 | 2002/2 | 2002/2 |
| Buprenorphine HCl | 2.16 | 2.16 | 2.16 | 2.16 | 2.16 | 2.16 |
| Lactose monohydrate | 20 | 20 | 20 | 20 | 20 | 20 |
| Corn starch | 10 | 10 | 10 | 10 | 10 | 10 |
| Povidone | 3 | 3 | 3 | 3 | 3 | 3 |
| Mannitol | 55.84 | 55.34 | 50.84 | 55.24 | 50.74 | 55.74 |
| Anhydrous citric acid | 4 | 4 | 4 | 4 | 4 | 4 |
| Sodium citrate | 4 | 4 | 4 | 4 | 4 | 4 |
| Ascorbic acid | / | 0.5 | 5 | 0.5 | 5 | / |
| Edetic acid (EDTA) | / | / | / | 0.1 | 0.1 | 0.1 |
| Magnesium | 1 | 1 | 1 | 1 | 1 | 1 |
| stearate | | | | | | |
| TOTAL | 100 | 100 | 100 | 100 | 100 | 100 |

The manufacturing process of each composition is the same i.e. a wet granulation of buprenorphine HCl. The manufacturing process used for those batches is the following : Mix lactose 450 mesh, maize starch, mannitol, povidone (first fraction), citric acid anhydrous, sodium citrate, ascorbic acid, EDTA (when used) in a planetary mixer. Dissolve buprenorphine HCl and povidone (second fraction) in ethanol. Granulate the mass by adding the ethanolic mixture. Once the mass is adequately granulated, dry it in an oven at 50°C during 5 hours. The dried granulated mass is then sieved on a 1 mm sieve. The granulate is then mixed with the lubricant (magnesium stearate) in a planetary mixer. Perform the tabletting of the mass using an automatic tabletting machine. The tablets are then filled into aluminium / PVC blisters.

All the batches were tested for stability at 40°C/75% RH and analyzed at T0 and T₁ₘₒₙₜₕ. All the impurities were detected and quantified. The method of quantification is the HPLC method described in the European Pharmacopoeia (4th ed., 2004) for buprenorphine HCl.

The results of the stability studies are given in table3

**Table 3 : stability data obtained on various compositions of buprenorphine hydrochloride at TO and after 1 month of storage at 40°C / 75 % relative humidity**

| | 20K | 21K | 22K | 25K | 26K | 27K |
|---|---|---|---|---|---|---|
| | 2002/2 | 2002/2 | 2002/2 | 2002/2 | 2002/2 | 2002/2 |
| | T0 | T0 | T0 | T0 | T0 | T0 |
| 10-keto-buprenorphine | 0,345 | 0,07 | 0,057 | 0,008 | 0,09 | 0.122 |
| total impurities | 0,807 | 0,369 | 0,387 | 0,205 | 0,285 | 0.342 |
| aspect of tablets | white | white | white | white | white | white |
| | T | T | T | T | T | T |
| | 1 month | 1 month | 1 month | 1 month | 1 month | 1 month |
| 10-keto-buprenorphine | 1,091 | 0,103 | 0,096 | 0,090 | 0,0105 | 1,616 |
| total impurities | 2,004 | 0,748 | 1,223 | 0,386 | 0,526 | 2,016 |
| aspect of tablets | white | light | coloured | white | coloured | white |
| | | coloured | spots | | spots | |
| | | spots | | | | |

The results of the stability studies performed on the batches of buprenorphine tablets described in table 2 give different indications. First, the instability of buprenorphine is confirmed when no antioxidant is added to the composition (the level of total impurities is of 2.004 % after 1 month storage at 40°C / 75 % RH). Secondly, the presence of an antioxidant, i.e. ascorbic acid in the present case, allow well, as described by Valenti and al in US patent 6,365,596 to stabilize oral compositions of buprenorphine since the amount of 10-keto-buprenorphine and total impurities are significantly lower in the compositions containing the antioxidant (the level of total impurities is respectively of 0.748 % and 1.223% for batches 21 K2002/2 and 22K2002/2 after 1 month storage at 40°C / 75 % RH) than in this not containing the antioxidant (20K2002/2). The protection to degradation provided by ascorbic acid is observed at relatively low concentrations (molar ratio bewteen ascorbic acid and buprenorphine is of 0.66) and is not greater when the concentration are increased (molar ratio between ascorbic acid and buprenorphine is of 6.6).
Furthermore, an optimal effect of ascorbic acid is observed below the ratio of concentrations between ascorbic acid and buprenorphine described in US patent 6,365,596. Indeed, US 6,365,596 describe a molar ratio between the antioxidant and buprenorphine comprised between 1:1 and 3:1. In the present invention, an optimal protective effect is obtained at a molar ratio between the antioxidant and buprenorphine lower than 1:1, advantageously lower than 0.9, preferably lower than 0.8, most preferably lower than 0.7, especially about 0.66. For example said molar ratio antioxidant / buprenorphine is comprised between 0.1 and 1.
Futhermore, an additive protective effect on buprenorphine is observed when a low concentration in ascorbic acid is combined with low concentrations of a chelating agent i.e. edetic acid (EDTA) in the composition (25K2002/2). Indeed, the formulations containing both ascorbic acid and edetic acid show a significanlty lower level in 10-keto-buprenorphine and in total impurities than the compositions containing only the antioxidants (the level of total impurities is of 0.386 % after 1 month storage at 40°C / 75 % RH). Both stabilizing agents seem to act synergistically to stabilize the buprenorphine in the compositions. It should also be noted that the use of EDTA only (without antioxidant) result in composition (27K2002/2) with very poor stability. The compositions containing the higher doses of ascorbic acid (22K2002/2 and 26k2002/2) present a relatively good impurity profile but the aspect of the tablets is not acceptable since coloured spots appear on the surface of the tablets probably due to the degradation of ascorbic acid itself. It should also be observed that the composition containing low concentration of ascorbic acid (21K2002/2) presented light coloured spots while the batch containing low concentration of ascorbic acid and edetic acid is still white after 1 month storage at 40°C / 75 % RH. The presence of edetic acid in the composition appears to somewhat reduce the degradation process of ascorbic acid.

### Example 2

This example describe the manufacturing of stable tablets of buprenorphine hydrochloride using the direct compression process
Mix 40g of lactose 450 mesh, 10g of maize starch, 40g of mannitol, 3g of povidone, 4g of citric acid anhydrous, 4g of sodium citrate, 0.5g of ascorbic acid, 0.1g of EDTA and 2.16 g of buprenorphine HCl in a planetary mixer..

Once the blend is homogeneous, proceed to direct compression and packaging of the tablets.

### Example 3

This composition describe the manufacturing of stable microgranules (also called beads or pellets) of buprenorphine hydrochloride. The manufactuirng process used is a three steps granulation extrusion and spheronisation of a plastic mass to obtain spherical granules with a diameter comprised between 0.7 and 1.4 mm. The granules can then optionally be coated.
Mix 80g of cellulose microcrystalline, 10g of sucrose stearate, 4g of citric acid, 0.5g of ascorbic acid and 0.1g of EDTA together in a planetary mixer. Dissolve 2.16g buprenorphine hydrochloride in a 50/50 (w/w) water/ alcohol mix. Begin to granulate the mass which is the planetary mixer with the 50/50 (w/w) water/ alcohol mix containing buprenorphine. Proceed to granulation until the mass acquires the required plastic properties. Extrudate the mass in an axial extruder. Spheronize the extrudates obtained on a spheronizer. The uncoated microgranules obtained are dried at 60 °C for 16 hours and then sieved between 0.7-1.4 mm. The microgranules can optionally been coated with for instance a protective coating or with a sustaine release coating like coating containing acrylic or cellulosic polymers. The microgranules are then filled into capsules and packaged.

### Example 4

This example describes different stable tablets of buprenorphine hydrochloride obtained by wet granulation (same manufacturing process as for example 1). All formulations combined an antioxidant and a chelating agent.

| Ingredients | 01 L02/1 | 01 L02/2 | 02L02/1 | 02L02/2 |
|---|---|---|---|---|
| Buprenorphine HCl | 2.16 | 2.16 | 2.16 | 2.16 |
| Lactose | 20 | 20 | 20 | 20 |
| monohydrate | 10 | 10 | 10 | 10 |
| Corn starch | 3 | 3 | 3 | 3 |
| Povidone | 55.84 | 55.84 | 55.84 | 55.84 |
| Mannitol | 4 | 4 | 4 | 4 |
| Anhydrous citric acid | 4 | 4 | 4 | 4 |
| Sodium citrate | 0.1 | 0.2 | / | / |
| Vitamine E | / | / | 0.05 | 0.01 |
| propylgallate | 0.5 | 0.1 | 0.5 | 0.1 |
| Edetic acid (EDTA) | 1 | 1 | 1 | 1 |
| Magnesium stearate | | | | |

## Claims

1. An oral pharmaceutical stable composition containing as active ingredient buprenorphine or a salt thereof, an antioxidant and a chelating agent, in which the molar ratio between said antioxidant and buprenorphine is strictly inferior to 1:1.

2. The oral composition of claim 1, wherein the concentration of the chelating agent with respect with the total composition is between 0.01 % and 5 % (weight by weight), advantageously between 0.05% and 3%.

3. The oral composition of claim 1, wherein said antioxidant is chosen among ascorbic acid or derivatives, vitamin E or derivatives, butylhydroxyanisole, butylhydroxytoluene, metabisulphite, and mixtures thereof.

4. The oral composition of claim 1, wherein said chelating agents is chosen among edetic acid, malic acid and mixtures thereof.

5. The oral composition of claim 1, wherein said antioxidant is ascorbic acid and said chelating agent is edetic acid

6. The oral composition of claim 1, wherein said oral composition is a tablet, a sublingual tablet, a coated tablet,

7. The oral composition of claim 1, wherein said oral composition is a capsule containing coated or uncoated microgranules or a capsule containing a powder

8. The oral composition of claim 7, wherein said oral composition is manufactured by a wet granulation process

9. The oral composition of claim 8, wherein said oral composition obtained by a wet granulation process and wherein the granulation liquid is a alcohol or hydro alcoholic mix

10. The oral composition of claim 1, wherein the molar ratio between said antioxidant and buprenorphine is comprised between 1:100 and 1:1.5

11. The oral composition of claim 1, wherein the composition is a sublingual tablet wherein the antioxidant is ascorbic acid and the chelating agent is edetic acid and wherein the molar ratio between ascorbic acid and buprenorphine is comprised between 1:100 and 1:1.5

12. The oral composition of claim 1, wherein the composition further contains a second active ingredient

13. The oral composition of claim 12, wherein the second active ingredient is also analgesic drug

14. The oral composition of claim 12, wherein the second active ingredient is an opioid antagonist.

15. The oral composition of claim 1, in which the molar ratio chelating agent(s)/antioxidant(s) is comprised between 0.05 and 20, preferably between 0.1 and 15, most preferably between 0.1 and 5.

## Patentansprüche

1. Stabile orale pharmazeutische Zusammensetzung, die als Wirkstoff Buprenorphin oder ein Salz davon, ein Antioxidans und einen Chelatbildner enthält, wobei das Molverhältnis zwischen dem Antioxidans und Buprenorphin grundsätzlich kleiner 1:1 ist.

2. Orale Zusammensetzung nach Anspruch 1, wobei die Konzentration des Chelatbildners in Bezug auf die gesamte Zusammensetzung zwischen 0,01 % und 5% (auf das Gewicht bezogen) liegt, vorteilhaft zwischen 0,05% und 3%.

3. Orale Zusammensetzung nach Anspruch 1, wobei das Antioxidans aus Ascorbinsäure oder deren Derivaten, Vitamin E oder dessen Derivaten, Butylhydroxyanisol, Butylhydroxytoluol, Metabisulfit und Gemischen davon ausgewählt ist.

4. Orale Zusammensetzung nach Anspruch 1, wobei der Chelatbildner aus Edetinsäure, Äpfelsäure und Gemischen davon ausgewählt ist.

5. Orale Zusammensetzung nach Anspruch 1, wobei das Antioxidans Ascorbinsäure ist und der Chelatbildner Edetinsäure ist.

6. Orale Zusammensetzung nach Anspruch 1, wobei die orale Zusammensetzung eine Tablette, eine sublinguale Tablette, eine überzogene Tablette ist.

7. Orale Zusammensetzung nach Anspruch 1, wobei die orale Zusammensetzung eine Kapsel ist, die überzogene oder nicht überzogene Mikrogranula enthält, oder eine Kapsel ist, die ein Pulver enthält.

8. Orale Zusammensetzung nach Anspruch 7, wobei die orale Zusammensetzung durch einen Nassgranulierungsprozess hergestellt wird.

9. Orale Zusammensetzung nach Anspruch 8, wobei die orale Zusammensetzung durch einen Nassgranulierungsprozess erhalten wird und wobei die Granulierungsflüssigkeit eine Alkohol- oder Hydroalkoholmischung ist.

10. Orale Zusammensetzung nach Anspruch 1, wobei das Molverhältnis zwischen dem Antioxidans und Buprenorphin zwischen 1:100 und 1:1,5 liegt.

11. Orale Zusammensetzung nach Anspruch 1, wobei die Zusammensetzung eine sublinguale Tablette ist, wobei das Antioxidans Ascorbinsäure ist und der Chelatbildner Edetinsäure ist, und wobei das Molverhältnis zwischen dem Antioxidans und Buprenorphin zwischen 1:100 und 1:1,5 liegt.

12. Orale Zusammensetzung nach Anspruch 1, wobei die Zusammensetzung des Weiteren einen zweiten Wirkstoff enthält.

13. Orale Zusammensetzung nach Anspruch 12, wobei der zweite Wirkstoff auch ein Analgetikum ist.

14. Orale Zusammensetzung nach Anspruch 12, wobei der zweite Wirkstoff ein Opioid-Antagonist ist.

15. Orale Zusammensetzung nach Anspruch 1, wobei das Molverhältnis zwischen dem oder den Chelatbildnern und dem Antioxidans oder den Antioxidantien zwischen 0,05 und 20, vorzugsweise zwischen 0,1 und 15, insbesondere zwischen 0,1 und 5 liegt.

## Revendications

1. Composition pharmaceutique orale stable contenant, comme ingrédient actif, de la buprénorphine ou un sel de celle-ci, un antioxydant et un agent de chélation, dans laquelle le rapport molaire entre ledit antioxydant et la buprénorphine est strictement inférieur à 1 : 1.

2. Composition orale selon la revendication 1, dans laquelle la concentration de l'agent de chélation par rapport à la composition totale est entre 0,01% et 5% (en poids), de manière avantageuse entre 0,05% et 3%.

3. Composition orale selon la revendication 1, dans laquelle ledit antioxydant est choisi parmi l'acide ascorbique ou ses dérivés, la vitamine E ou ses dérivés, le butylhydroxyanisole, le butylhydroxytoluène, le métabisulfite et leurs mélanges.

4. Composition orale selon la revendication 1, dans laquelle ledit agent de chélation est choisi parmi l'acide édétique, l'acide malique et leurs mélanges.

5. Composition orale selon la revendication 1, dans laquelle ledit antioxydant est l'acide ascorbique et ledit agent de chélation est l'acide édétique.

6. Composition orale selon la revendication 1, dans laquelle ladite composition orale est un comprimé, un comprimé sublingual, un comprimé enrobé.

7. Composition orale selon la revendication 1, dans laquelle ladite composition orale est une capsule contenant des microgranulés enrobés ou non enrobés ou une capsule contenant une poudre.

8. Composition orale selon la revendication 7, dans laquelle ladite composition orale est fabriquée par un procédé de granulation par voie humide.

9. Composition orale selon la revendication 8, dans laquelle ladite composition orale est obtenue par un procédé de granulation par voie humide et dans laquelle le liquide de granulation est un alcool ou un mélange hydro-alcoolique.

10. Composition orale selon la revendication 1, dans laquelle le rapport molaire entre ledit antioxydant et ladite buprénorphine est compris entre 1 : 100 et 1 : 1,5.

11. Composition orale selon la revendication 1, dans laquelle la composition est un comprimé sublingual dans lequel l'antioxydant est l'acide ascorbique et l'agent de chélation est l'acide édétique et dans laquelle le rapport molaire entre l'acide ascorbique et la buprénorphine est compris entre 1 : 100 et 1 : 1,5.

12. Composition orale selon la revendication 1, dans laquelle composition contient en outre un second ingrédient actif.

13. Composition orale selon la revendication 12, dans laquelle le second ingrédient actif est également un médicament analgésique.

14. Composition orale selon la revendication 12, dans laquelle le second ingrédient actif est un antagoniste opioïde.

15. Composition orale selon la revendication 1, dans laquelle le rapport molaire agent(s) de chélation/antioxydant(s) est compris entre 0,05 et 20, de préférence entre 0,1 et 15, et de manière préférée entre toutes entre 0,1 et 5.
